# FASCICULE DE BREVET EUROPEEN

(11) **EP 1 894 892 B1**
(45) Date de publication et mention de la délivrance du brevet: **28.03.2012**
(21) Numéro de dépôt: 07009770.4
(22) Date de dépôt: 16.05.2007
(51) Int. Cl.: A01K 67/033, C05F 17/00, C05F 17/02

(54) **Procédé de traitement de déchets organiques et dispositif pour sa mise en oeuvre**
Verfahren zur Behandlung von organischen Abfällen und Vorrichtung zur Umsetzung dieses Verfahrens
Method of processing organic waste and device for implementing same

(30) Priorité: 30.08.2006 CH 13972006
(43) Date de publication de la demande: 05.03.2008
(73) Titulaire: Meusy, Guy, 1296 Coppet (CH)
(72) Inventeur: Meusy, Guy, 1296 Coppet (CH)
(74) Mandataire: Micheli & Cie SA

(56) Documents cités:
- WO-A-00/43329
- WO-A-94/19296
- WO-A-2004/043879
- LU-A1- 85 188
- US-A- 5 441 552
- US-A1- 2003 059 931
- JULIE WEISENHORN: "Vermiculture: Promote Global Worming!"[Online] décembre 2000 (2000-12), pages 1-13, XP002494268 Department of Horticultural Science, University of Minnesota Extrait de l'Internet: URL:www.sustland.umn.edu/maint/docs/Vermic ulture.doc> [extrait le 2008-09-02]

## Description

La présente invention est relative au domaine du traitement des déchets organiques en vue de leur revalorisation, en particulier aux procédés d'obtention de composts. L'invention a pour objet un procédé de traitement et de recyclage des déchets organiques en vue d'obtenir du terreau ou du compost. Le procédé objet de l'invention est utilisable pour tous types de déchets organiques, tels que des déchets ménagers, des déchets verts, des boues d'épuration ou de décantation des eaux ou des résidus de produits laitiers, et le fumier d'élevage.

L'invention a également pour objet un dispositif pour la mise en oeuvre du procédé de traitement.

Le compostage est un procédé biologique contrôlé de conversion et de valorisation des substances organiques par décomposition des déchets organiques hétérogènes fermentescibles.

L'indispensable humus, communément appelé « Terre végétale » est constitué de matière organique décomposée ou en cours de décomposition. Le compostage reproduit le processus naturel d'humification, c'est-à-dire la décomposition puis la stabilisation des matières organiques sous l'effet de facteurs biologiques. Mis en oeuvre par l'homme, ce processus est plus rapide et permet d'obtenir de meilleurs résultats et de meilleurs rendements que lorsqu'il se réalise naturellement.

Dans toute masse de déchets organiques, d'origine animale ou végétale, des micro-organismes existent, se développent et se multiplient rapidement utilisant l'humidité et l'oxygène contenus dans la masse pour leur métabolisme en se nourrissant de matière organique. Ils élaborent leur protoplasme cellulaire en profitant d'une partie du carbone, dont la plus grande part leur sert de source d'énergie ; elle est brûlée et rejetée par leur respiration sous forme de gaz carbonique.

Ainsi par ce phénomène, les micro-organismes élèvent la température de la masse de déchets organiques jusqu'à des températures de l'ordre de 70 degrés, parfois plus. De ce fait, on assiste au remplacement des bactéries mésophiles utiles mais parfois pathogènes pour l'homme, par des bactéries thermophiles qui se développent dans les meilleures conditions à des températures comprises entre 45 et 65 degrés Celsius. A ce niveau de température apparaissent alors des actinomycètes et des champignons thermophiles qui prennent leur importance au cours des phases finales du compostage en particulier en s'attaquant aux substances riches en cellulose, telles que le papier, les écorces etc. Cette activité des micro-organismes se vérifie par une diminution substantielle du volume du tas de déchets initial.

L'intervention humaine au cours du processus de compostage est essentielle afin d'optimiser tous les facteurs nécessaires et indispensables au développement des micro-organismes sans lesquels rien n'est possible. Cette intervention humaine peut prendre plusieurs formes pour optimiser le procédé naturel de décomposition, il s'agit entre autre d'opérations telles que : broyage des déchets, équilibrage des mélanges en éléments nutritifs, homogénéisation, humidification, aération, isolement ou refroidissement des volumes traités. Un compost obtenu en tenant compte de ces données sera d'une excellente qualité et très rapidement utilisable.

Le document US 2003/0059931 décrit un dispositif pour le compostage de matière organique comprenant une pluralité de tiroirs dont le fond est percé de trous agencés dans un container. Chaque tiroir contient une litière comprenant des vers. La récolte du compost se fait par le fond de chaque tiroir.

Le document LU 85 188 décrit un dispositif pour le compostage comprenant un élément inférieur ayant un fond amovible, un élément supérieur et un élément séparateur du type grille. La récolte du compost se fait par le fond amovible de l'élément inférieur, l'élément séparateur retenant les vers et la nouvelle matière organique à décomposer.

Le document WO 2004/043879 décrit un dispositif pour le compostage comprenant un conteneur muni d'un couvercle et comprenant une structure microporeuse ne présentant pas d'ouverture, un vide d'air étant présent entre les parois du conteneur et de la structure microporeuse.

Actuellement, les déchets organiques, par exemple les déchets verts, sont récupérés puis amenés vers des lieux de stockage à ciel ouvert. Ils sont ensuite broyés à l'aide de machines adéquates de manière à les calibrer et à obtenir un mélange homogène, puis ils sont répartis sur des plates-formes appelées andins qui présentent des dimensions de l'ordre de deux à trois mètres de large pour une longueur pouvant atteindre une cinquantaine de mètres. Ces andins accueillent les déchets broyés sur une hauteur de l'ordre de 2 à 3 mètres. Ces volumes de déchets sont ensuite travaillés de manière à aérer et à humidifier les tas car la fermentation naturelle provoque une augmentation substantielle de la température. Ces opérations doivent se poursuivre pendant plusieurs mois de manière à ce que sous l'effet de la fermentation naturelle le volume des tas diminuent. Le compost arrivé à maturité, après une période de l'ordre d'une année, est ensuite tamisé pour être vendu comme terreau végétal. Les composts obtenus selon ces procédés traditionnels ne sont en général pas de très bonne qualité et ne trouvent que difficilement acquéreur. Il est alors nécessaire d'éliminer ce compost en le brûlant par exemple. Certes les volumes sont moindres qu'au départ, mais les procédés existant nécessitent d'une part, de grandes surfaces de stockage pour former les andins, et d'autre part, une main d'oeuvre importante pour manipuler les tas, les aérer, les retourner et contrôler leur température. Par ailleurs, selon les procédés connus, le temps nécessaire pour obtenir un produit dont la qualité n'est de loin pas optimale, est de l'ordre d'une dizaine de mois à une année.

Le but de la présente invention est de proposer un procédé de traitement des déchets qui obvie aux inconvénients mentionnés ci-dessus en raccourcissant significativement le temps d'obtention du produit fini, qui permet de réduire la main d'oeuvre nécessaire à son traitement et qui offre un produit fini de grande qualité.

Un autre but de l'invention est de permettre la réduction d'environ deux tiers du volume de la matière initiale à l'issue du traitement selon le procédé objet de l'invention.

Un autre but de l'invention consiste à offrir un dispositif pour la mise en oeuvre du procédé de traitement.

Ces buts sont atteints par un procédé qui se distingue par les étapes mentionnées à la revendication 6, respectivement par un dispositif pour la mise en oeuvre du procédé qui se distingue par les caractéristiques énoncées à la revendication 1.

Des modes de réalisation particuliers de l'invention sont définis dans les revendications dépendantes.

D'autres caractéristiques et avantages de la présente invention apparaîtront à la lecture de la description détaillée suivante faite en référence aux dessins annexés dans lesquels :
- la figure 1 est une vue éclatée en perspective de deux modules adjacents du dispositif pour le traitement des déchets selon le procédé objet de l'invention.
- La figure 2 est une vue en perspective de la partie supérieure ou élément de transfert du dispositif selon l'invention.
- La figure 3 est une vue de côté de deux modules adjacents tels qu'illustrés à la figure 1 munis de l'élément de transfert représenté à la figure 2.

Le procédé selon l'invention va tout d'abord être décrit en référence aux figures puis suivra une description plus détaillée du dispositif pour la mise en oeuvre du procédé.

Les premières étapes de traitement des déchets organiques sont identiques à celles réalisées dans les procédés usuels. Les déchets une fois collectés, sont tout d'abord broyés à l'aide de machines adéquates. Cette étape de broyage ou de défibrisation des matériaux est en effet favorable puisqu'elle augmente la surface d'attaque de la matière par les micro-organismes d'où une décomposition naturelle accélérée. Le broyage provoque par ailleurs une homogénéisation de la masse à composter et partant une décomposition plus uniforme. Enfin, il assure une meilleure organisation et un réchauffement plus homogène de la masse à traiter. Les étapes de traitement suivantes diffèrent des procédés traditionnels en ce que la matière broyée et pré-compostée n'est pas étendue sur des andins et travaillée comme expliqué dans l'introduction de la présente demande.

Pour accélérer et optimiser le procédé de traitement de cette matière de base, on fait appel à des vers d'élevage qui vont contribuer à la décomposition accélérée des déchets organiques.

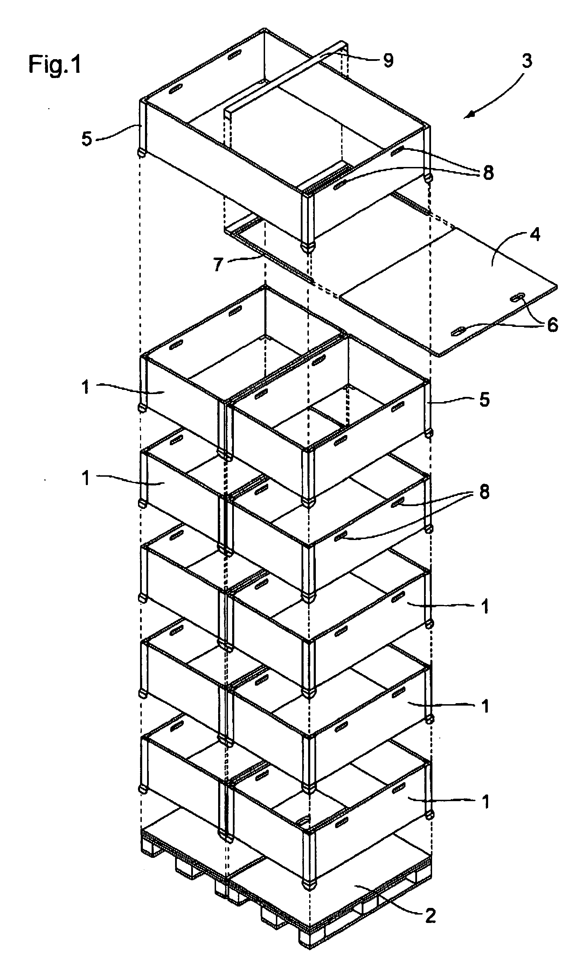

Les vers d'élevage sont parfois utilisés pour augmenter la rapidité de la décomposition de la matière organique. Toutefois, dans les installations de traitement conventionnelles, il est difficile de maîtriser l'adjonction de vers dans les andins car les vers ne sont pas confinés dans une structure fermée et peuvent en conséquence s'échapper. Le procédé objet de l'invention consiste à passer d'un traitement de type horizontal par étalage de la matière sur des andins à un traitement vertical et confiné des déchets. A cet effet, on va utiliser une structure telle qu'illustrée à la figure 1 qui se compose de modules constitués d'un empilement de plusieurs cadres 1 agencés les uns au-dessus des autres. Ces modules sont de préférence constitués de 4 à 6 cadres 1 superposés, chaque cadre 1 présente des dimensions de l'ordre de 120 cm par 80 cm pour une hauteur d'environ 40 cm. Les cadres 1 comprennent 4 panneaux généralement réalisés en bois et maintenus par des équerres 5 en acier ou en tout autre matériau présentant les caractéristiques de rigidité et de solidité requises.

Les cadres 1 sont disposés sur un support 2 de type palette qui facilite le déplacement d'un module entier à l'aide d'un élévateur classique. Plusieurs modules composés de 4 à 6 cadres 1 superposés sont disposés côte à côte de préférence dans un hangar ou sous abri.

La première étape de traitement selon le procédé objet de l'invention consiste à placer dans le premier cadre 1 formant la partie basse d'un module, une litière contenant des vers. Cette litière est constituée d'un substrat organique pré-composté qui sert à la fois de nourriture et d'habitat aux vers.

Les vers utilisés à cet effet sont issus de la famille des lombricoïdes et l'espèce préférée est celle de l'Eisenia foedita andrei qui s'accommode d'écarts de température compris entre 0 et 30 degrés Celsius. De plus, des températures de l'ordre de 22 à 27 degrés, qui sont fréquentes dans les tas de matière organique en décomposition, favorisent la croissance et la multiplication des vers de cette espèce. La litière qui sert à la fois d'habitat et de nourriture aux vers comprendra de préférence une densité de vers comprise entre 70 et 90 vers, de préférence 80 vers par kilo de matière organique. Typiquement, les litières utilisées dans le cadre inférieur 1 comprennent une population d'environ 100'000 vers. Une fois la litière installée dans le cadre inférieur du module, on charge par strates successives, de la matière pré-compostée à traiter jusqu'à atteindre le niveau supérieur du module de cadres. Durant cette opération, on contrôle régulièrement la température et le degré d'humidité de la matière organique. A une température favorable comprise entre 22 et 28 degrés, les vers connaissent une croissance rapide et se multiplient. Quatre à six semaines suffisent à ce qu'un vers nouveau-né atteigne sa taille adulte et possède toutes ses facultés reproductrices. Le degré d'humidité de la matière organique est également important, car ainsi la matière pré-compostée introduite dans le module, est directement accessible aux vers lorsque l'on est en présence d'un taux d'humidité voisin de 85%. On veillera en conséquence à arroser la matière organique à traiter de manière à maintenir un taux d'humidité compris entre 75 et 95%, de préférence 85% et une température comprise entre 20 et 28 degrés, de préférence 25 degrés. Dans ces conditions de température et d'humidité, les vers croissent et se multiplient rapidement et puisent leur nourriture dans les matières organiques pré-compostées qu'ils transforment rapidement en un compost ou terreau directement utilisable par exemple comme engrais vert ou naturel dans l'agriculture ou comme amendement organique. L'arrosage du module par le haut est effectué avec de l'eau ou de préférence à l'aide de lixiviat (jus issu de la décomposition des végétaux et de l'eau) qui optimise encore le processus de fermentation.

Une fois que les strates de matière organique ont atteint le sommet du dernier cadre 1 du module, on dispose au-dessus de ce dernier, un dispositif de transfert 3 qui va permettre de déplacer les vers présents dans le premier module de cadres 1 vers un second module adjacent au premier.

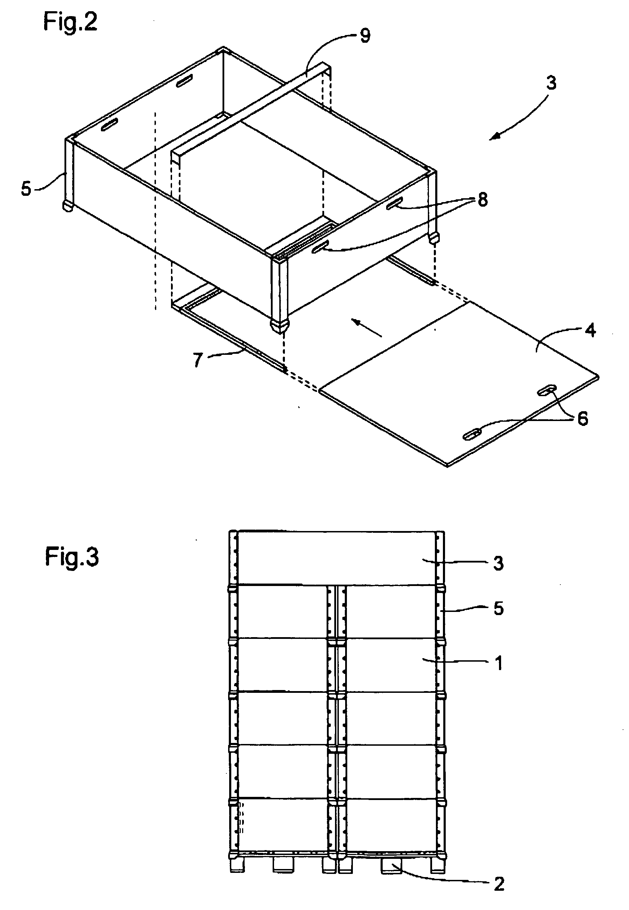

Le dispositif de transfert 3, illustré à la figure 2, se présente sous la forme d'un cadre similaire à ceux qui constituent le module de cadres 1, à la différence près qu'il présente une longueur double de manière à pouvoir s'adapter au sommet de deux colonnes adjacentes de cadres 1. L'élément de transfert 3 est en outre pourvu dans sa partie basse et sur une moitié de sa longueur, d'un panneau 4 formant un fond qui peut coulisser dans une glissière 7 aménagée dans la partie inférieure de l'élément de transfert 3. Le panneau 4 formant le fond ne s'étend que sur la moitié de la surface de l'élément de transfert 3. Des découpes 6 agencées à l'une des extrémités du panneau de fond 4 rendent son utilisation plus aisée. Dans une variante, le fond 4 peut être monté sur des charnières de manière à pouvoir basculer. Avantageusement, le module de transfert 3 comportera un ou plusieurs raidisseurs 9 en acier galvanisé par exemple.

Dans un mode préféré du dispositif pour la mise en oeuvre du procédé selon l'invention, les panneaux avant et arrière des cadres 1 et de l'élément de transfert 3 sont également pourvus de découpes 8 formant poignées pour faciliter leur manipulation.

Lorsque la matière organique atteint le sommet du dernier cadre 1 du premier module, on dispose l'élément de transfert 3 sur le sommet de deux modules de cadres 1 adjacents, puis on dispose de la matière organique dans le fond 4 de l'élément de transfert 3 qui obture le module de cadre 1 qui n'a pas encore été traité. Dans le premier module, la matière est arrivée à maturation et ne présente en conséquence plus les qualités nutritives que les vers apprécient. Dans un laps de temps d'environ 24 heures, les vers vont se déplacer pour atteindre la matière organique située sur le panneau coulissant 4 formant le fond de l'élément de transfert 3. Il suffit ensuite de faire coulisser le panneau 4 formant le fond de l'élément de transfert 3 pour que les vers se déversent dans le second module préalablement chargé avec de la matière organique pré-compostée.

On retire ensuite l'élément de transfert 3 et le procédé de charge en strates successives de matière à traiter est ensuite répété dans le second module.

Grâce à ce procédé de traitement des matières organiques dans lequel la matière organique est disposée verticalement dans une pluralité de modules agencés les uns à côté des autres, la manutention est grandement réduite par rapport aux procédés de traitement horizontaux. En effet, une fois un module de cadres 1 traité, il peut être déplacé et déchargé avec un simple élévateur puis remis en place à côté d'un module en cours de traitement. Un personnel réduit suffit à la mise en oeuvre de ce procédé qui est adapté pour traiter des volumes de l'ordre de dix mille tonnes de déchets organiques par an.

Avec les procédés traditionnels dans lesquels la matière organique est simplement entreposée horizontalement sur des andins et transformée par décomposition ou dégradation biologique naturelle, il faut environ 12 mois pour obtenir un compost ou terreau utilisable. Selon le procédé objet de l'invention, deux à trois mois suffisent pour obtenir le même résultat avec en plus un produit fini qui présente des qualités organiques nettement supérieures. On notera en effet que les vers neutralisent entre autres les métaux lourds présents dans la matière organique à traiter.

Grâce au procédé de traitement selon l'invention, le volume de matière à traiter diminue d'environ deux tiers par rapport au volume de déchet initial ce qui est nettement supérieur à la diminution de volume obtenue par les procédés traditionnels.

## Revendications

1. Dispositif pour le traitement de déchets organiques en vue de leur revalorisation comportant une pluralité de modules constitués de cadres sans fond (1) superposés, les modules étant agencés les uns à côté des autres sur des supports facilitant leur transport **caractérisé en ce qu'**il comporte en outre un élément de transfert (3) dimensionné de manière à s'adapter au sommet de deux modules adjacents, et **en ce que** ledit élément de transfert est muni sur la moitié de sa surface d'un fond amovible (4) mobile par rapport à l'élément de transfert (3) entre une première position dans laquelle ledit fond amovible n'obture que l'un des deux modules adjacents et une seconde position dans laquelle ledit un des deux modules n'est plus totalement obturé .

2. Dispositif selon la revendication 1, **caractérisé en ce que** le fond amovible (4) est monté coulissant par rapport à l'élément de transfert dans une glissière (7) agencée dans la partie basse de l'élément de transfert (3).

3. Dispositif selon la revendication 2, **caractérisé en ce que** le fond (4) de l'élément de transfert (3) coulissant comporte deux découpes (6) formant des poignées.

4. Dispositif selon la revendication 1, **caractérisé en ce que** le fond (4) de l'élément de transfert (3) est monté basculant sur des charnières.

5. Dispositif selon l'une des revendications 1 à 4 **caractérisé en ce que** les panneaux avant et arrière des cadres (1) comportent des découpes (8) formant des poignées pour faciliter leur manutention.

6. Procédé de traitement de déchets organiques en vue de leur revalorisation mis en oeuvre par un dispositif selon l'une des revendications 1 à 5, **caractérisé en ce qu'**il comporte les étapes suivantes.
- introduction d'une litière de matière organique comprenant des vers dans le fond d'un premier module du dispositif,
- ajout par strates successives de matière organique pré-compostée dans ledit premier module,
- contrôle de la température et du degré d'humidité de la matière en décomposition dans le premier module et adjonction de liquide de manière à maintenir une température et un taux d'humidité appropriés au vermicompostage,
- installation d'un élément de transfert (3) du dispositif comprenant un fond (4) amovible au sommet du premier module et d'un second module du dispositif adjacent au premier, le fond (4) dudit élément de transfert étant placé dans une première position obturant le second module de cadre, le premier module de cadre n'étant pas couvert,
- introduction de nouvelle matière organique sur le fond (4) du module de transfert (3) dans sa première position obturant le second module de cadre,
- escamotage du fond (4) de l'élément de transfert pour le placer dans une seconde position où le second module n'est plus totalement couvert, et permettant ainsi de transférer dans le second module de cadre les vers précédemment contenus dans le premier module de cadre et s'étant déplacés vers le haut dans la nouvelle matière organique disposée sur le fond (4) de l'élément de transfert (3).
- retrait de l'élément de transfert 3 et le procédé de charge en strates successives de matière à traiter est ensuite répété dans le module suivant.

7. Procédé de traitement de déchets organiques selon la revendication 6, **caractérisé en ce que** la litière utilisée comprend de 60 à 100 vers par kilo de matière organique, de préférence 80 vers par kilo de matière.

8. Procédé de traitement de déchets organiques selon l'une des revendications précédentes, **caractérisé en ce que** les vers utilisés appartiennent à l'espèce Eisenia foedita andrei.

9. Procédé de traitement de déchets organiques selon l'une des revendications précédentes, **caractérisé en ce que** les déchets organiques à traiter sont constitués principalement de déchets verts pré-compostés.

## Claims

1. Device for processing organic waste for the purpose of reuse, comprising a plurality of modules formed of superimposed bottomless frames (1), the modules being arranged one beside the other on supports facilitating their transportation, **characterised in that** it also comprises a transfer element (3) dimensioned so as to fit the top of two adjacent modules, and **in that** the said transfer element is provided on half of its surface with a removable base (4) which can move with respect to the transfer element (3) between a first position in which the said removable base closes only one of the two adjacent modules, and a second position in which the said one of the two modules is no longer totally closed.

2. Device as claimed in claim 1, **characterised in that** the removable base (4) is mounted so as to slide with respect to the transfer element in a slide (7) arranged in the lower part of the transfer element (3).

3. Device as claimed in claim 2, **characterised in that** the base (4) of the sliding transfer element (3) has two cut-outs (6) forming handles.

4. Device as claimed in claim 1, **characterised in that** the base (4) of the transfer element (3) is mounted so as to swivel on hinges.

5. Device as claimed in any one of claims I to 4, **characterised in that** the front and rear panels of the frames (1) have cut-outs (8) forming handles to facilitate handling thereof.

6. Method for processing organic waste for the purpose of reuse, implemented by a device as claimed in any one of claims 1 to 5, **characterised in that** it includes the following steps:
- introduction of organic material litter including worms in the bottom of a first module of the device,
- addition by successive strata of precomposted organic material into the said first module,
- monitoring of the temperature and degree of humidity of the decomposing material in the first module and addition of liquid so as to maintain a temperature and humidity level appropriate for vermicomposting,
- installation of a transfer element (3) of the device comprising a removable base (4) on the top of the first module and of a second module of the device adjacent to the first, the base (4) of the said transfer element being placed in a first position closing the second frame module, the first frame module not being covered,
- introduction of new organic material on the base (4) of the transfer module (3) in its first position closing the second frame module,
- retraction of the base (4) of the transfer element in order to place it in a second position where the second module is no longer totally covered, and thus permitting transfer into the second frame module of the worms previously contained in the first frame module and having moved upwards in the new organic material disposed on the base (4) of the transfer element (3),
- withdrawal of the transfer element 3 and the method of loading in successive strata of material to be processed is then repeated in the following module.

7. Method for processing organic waste as claimed in claim 6, **characterised in that** the litter used comprises from 60 to 100 worms per kilo of organic material, preferably 80 worms per kilo of material.

8. Method for processing organic waste as claimed in any one of the preceding claims, **characterised in that** the worms used belong to the species *Eisenia foedita andrei*.

9. Method for processing organic waste as claimed in any one of the preceding claims, **characterised in that** the organic waste to be processed is formed principally of precomposted green waste.

## Patentansprüche

1. Vorrichtung zur Behandlung von organischen Abfällen im Hinblick auf deren Wiederverwertung, wobei die Vorrichtung mehrere Module umfasst, die aus gestapelten Rahmen ohne Boden (1) bestehen, wobei die Module auf Gestellen, die ihren Transport erleichtern, nebeneinander angeordnet sind, **dadurch gekennzeichnet, dass** die Vorrichtung außerdem ein Transportelement (3) umfasst, das derart dimensioniert ist, dass es sich an den oberen Teil von zwei benachbarten Modulen anpasst, und **dadurch**, dass das Transportelement auf der Hälfte seiner Oberfläche mit einem herausnehmbaren Boden (4) versehen ist, der in Bezug auf das Transportelement (3) zwischen einer ersten Stellung, in der der herausnehmbare Boden nur eines der zwei benachbarten Module verschließt, und einer zweiten Stellung, in der das eine der zwei Module nicht mehr vollständig verschlossen ist, bewegt werden kann.

2. Vorrichtung nach Anspruch 1, **dadurch gekennzeichnet, dass** der herausnehmbare Boden (4) in Bezug auf das Transportelement verschiebbar in einer Laufschiene (7) montiert ist, die im unteren Teil des Transportelements (3) angeordnet ist.

3. Vorrichtung nach Anspruch 2, **dadurch gekennzeichnet, dass** der verschiebbare Boden (4) des Transportelements (3) zwei gestanzte Ausschnitte (6) umfasst, die Griffe bilden.

4. Vorrichtung nach Anspruch 1, **dadurch gekennzeichnet, dass** der Boden (4) des Transportelements schwenkbar auf Scharnieren montiert ist.

5. Vorrichtung nach einem der Ansprüche 1 bis 4, **dadurch gekennzeichnet, dass** die vorderen und die hinteren Platten der Rahmen (1) gestanzte Ausschnitte (8) umfassen, die Griffe bilden, um deren Handhabung zu erleichtern.

6. Verfahren zur Behandlung von organischen Abfällen im Hinblick auf deren Wiederverwertung, das mit einer Vorrichtung nach einem der Ansprüche 1 bis 5 durchgeführt wird, **dadurch gekennzeichnet, dass** das Verfahren die folgenden Schritte umfasst:
- Einbringen einer Streu aus organischem Material, das Würmer umfasst in den Boden eines ersten Moduls der Vorrichtung,
- Zugeben von vorkompostiertem organischem Material in das erste Modul in aufeinander folgenden Schichten,
- Steuern der Temperatur und des Feuchtigkeitsgrads des sich im ersten Modul zersetzenden Materials und Hinzufügen von Flüssigkeit, um eine Temperatur und einen Feuchtigkeitsgehalt, die für die Wurmkompostierung geeignet sind, aufrechtzuerhalten,
- Einsetzen eines Transportelements (3) der Vorrichtung, das am oberen Teil des ersten Moduls einen herausnehmbaren Boden (4) umfasst, und eines zweiten Moduls der Vorrichtung, benachbart zu dem ersten Modul, wobei der Boden (4) des Transportelements in einer ersten Stellung positioniert ist, wodurch er das zweiten Rahmenmodul verschließt, wobei das erste Rahmenmodul nicht abgedeckt ist,
- Einbringen von neuem organischem Material in den Boden (4) des Transportmoduls (3) in seiner ersten Stellung, wodurch er das zweite Rahmenmodul verschließt,
- Einziehen des Bodens (4) des Transportelements, um ihn in einer zweiten Stellung zu positionieren, in der das zweite Modul nicht mehr vollständig verschlossen ist und wodurch somit das Transportieren von Würmern, die zuvor in dem ersten Rahmenmodul enthalten waren, in das zweite Rahmenmodul ermöglicht wird und die Würmer in dem neuen organischen Material, das sich im Boden (4) des Transportelements (3) nach oben umgesiedelt werden,
- Zurückziehen des Transportelements (3) und das Verfahren zum Füllen mit zu behandelndem Material in aufeinander folgenden Schichten wird anschließend im folgenden Modul wiederholt.

7. Verfahren zur Behandlung von organischen Abfällen nach Anspruch 6, **dadurch gekennzeichnet, dass** die verwendete Streu 60 bis 100 Würmer pro Kilo organisches Material, vorzugsweise 80 Würmer pro Kilo Material umfasst.

8. Verfahren zur Behandlung von organischen Abfällen nach einem der vorhergehenden Ansprüche, **dadurch gekennzeichnet, dass** die verwendeten Würmer zur Spezies Eisenia foedita andrei gehören.

9. Verfahren zur Behandlung von organischen Abfällen nach einem der vorhergehenden Ansprüche, **dadurch gekennzeichnet, dass** die zu behandelnden organischen Abfälle hauptsächlich aus vorkompostierten grünen Abfällen bestehen.
